# EUROPEAN PATENT SPECIFICATION

(11) **EP 1 782 818 B1**
(45) Date of publication and mention of the grant of the patent: **30.09.2009**
(21) Application number: 06425217.4
(22) Date of filing: 29.03.2006
(51) Int. Cl.: A61K 35/74, A61K 31/80, A61P 1/06, A61P 1/14, A61P 1/00

(54) **Pharmaceutical composition comprising Bacillus Coagulans and symethicone for the treatment of gastrointestinal pathologies**
Pharmazeutische Zusammensetzung enthaltend Bacillus Coagulans und Simethicon zur Behandlung von gastrointestinalen Störungen
Compositions pharmaceutiques comprenant le Bacillus Coagulans et de la simethicone pour le traitement de troubles gastrointestinaux

(30) Priority: 26.10.2005 IT RM20050531
(43) Date of publication of application: 09.05.2007
(73) Proprietor: D.M.G. Italia Srl, 00040 Pomezia (RM) (IT)
(72) Inventor: Mercuri, Luigi, 00040 Pomezia (RM) (IT)
(74) Representative: Sarpi, Maurizio

(56) References cited:
- EP-A1- 1 022 023
- WO-A-20/05055934
- WO-A2-00/07606
- US-A1- 2004 185 032
- R. HARTEMINK: "What's in a name- Bacillus coagulans or Lactobacillus sporogenes?" INTERNET ARTICLE, [Online] 2006, XP002412615 Retrieved from the Internet: URL:www.food-info.net/uk/ff/sporogenes> [retrieved on 2006-12-19]
- SWEETMAN S C (ED): "Martindale, PASSAGE" 2002, MARTINDALE : THE COMPLETE DRUG REFERENCE, LONDON : PHARMACEUTICAL PRESS, GB, PAGE(S) 1249 , XP002296551 ISBN: 0-85369-499-0 * page 1, column 2 *
- ANONYMOUS: "Bactolac Pharmaceutical Pvt Ltd" REGISTRY OF BIOMEDICAL COMPANIES, [Online] 17 February 2005 (2005-02-17), XP002412616 Retrieved from the Internet: URL:http://web.archive.org/web/20050217094 251/http://hum-molgen.org/companies/profil e.php3/2427> [retrieved on 2006-12-21]
- SANDERS M.E., MORELLI L., TOMPKINS T. A: "Sporeformers as human probiotics: Bacillus, Sporolactobacillus, and Brevibacillus" COMPREHENSIVE REVIEWS IN FOOD SCIENCE AND FOOD SAFETY, vol. 2, 2003, pages 101-110, XP002412617

## Description

The present invention relates to a composition comprising Bacillus Coagulans (also sometimes called Lactobacillus Sporogenes) and a silicone emulsion (symethicone) that can be used for the treatment of nursling's intestinal disorders. In addition, the composition which is the object of the invention can be effectively used for the treatment of the gas colic.

As known enteralgias are due to the formation on intestinal gas and are not only associated with a shooting stomachache but also give often rise to alterations of the intestinal functions, possible diarrhoea or constipation and events connected to vitamin deficiency, reduced absorption of essential nutrients, and impoverishment of the endogenous intestinal, bacterial flora.

Intestinal disorders to which nurslings are subjected in the first three months of life are typical. A frantic fit of crying of nurslings is often due just to their suck of milk which causes an increase in the peristalsis and the formation of intestinal gas with the consequent onset of stomachache. These intestinal disorders hit both breast- and bottle-fed babies and in most cases subside as babies get at the age of three months.

Frequently, digestion problems connected to intolerance to lactose occur during childhood and give rise to bothersome intestinal disorders bringing to an impoverishment of the microorganisms of the intestinal, bacterial flora. Such microorganisms play an important role in the correct development of the digestion processes.

As previously mentioned, the unbalance of the endogenous flora brings to alterations of the intestinal function with possible diarrhoea or constipation with the consequence of stomachache and events connected to vitamin deficiency, reduced absorption of essential nutrients.

The object of the present invention is to provide a composition which is able, in one administering, to build up again the biological mechanical barrier formed naturally by intestinal saprophytes to inhibit the take of pathogenic bacterial species and at the same time to reduce the surface tension of the air bubbles which form in the gastric apparatus.

From W02005/055934 it is known a method for treating irritable bowel syndrome by increasing carbohydrate absorption by administering a composition containing a Bacillus coagulans bacterium wherein an anti-gas agent as simethicone can be included.

According to the present invention it is provided a composition including symethicone and Bacillus coagulans to treat nursling's intestinal disorders.

In particular, a synergy is reached by using Bacillus Coagulans, especially microencapsulated Bacillus Coagulans, dispersed in a silicone emulsion (symethicone).

Bacillus Coagulans was originally called Lactobacillus Sporogenes, and, despite being reclassified, is still often erroneously called L. Sporogenes. Bacillus Coagulans is an aerobic grampositive microorganism which produces lactic acid. It was described for the first time in 1933 and isolated in Japan in 1949. Typically, it is present in the intestine where it has a very important function in the digestion, since it synthesizes vitamin B3, B5, B6, B12, folic acid, biotine, vitamin K, and several digestive enzymes. Furthermore, unlike the strains of Lactobacillus, Bacillus coagulans is resistant to heat, and as in this case it is naturally microencapsulated, it also withstands to antibiotics and biliary acids and is stable to room temperature for three years.

Preferably, Bacillus Coagulans is used in the composition of the present invention when the spore concentration is between five hundred thousand and fifty thousand millions spores per gram, moreover, preferably when the spore concentration is fifty thousand millions spores per gram.

Symethicone belongs instead to the class of linear polydimethylsiloxanes which have been widely used in human medicine for several years.

It is the composition of the chemical class of siloxanes which is most used in therapies. Symethicone is formed by dimethicone to which silica gel is added to promote its dispersion in water; it is a surfactant acting as antifoaming agent which is not absorbed again by the digestion mucous membrane and its local antifoaming action is shown as it is used for radiography, echographia or endoscopy of the digestive tube. Furthermore, it has been shown that symethicone is able to inhibit the bacterial proliferation particularly of Helicobacter pilori. At last, the antifoaming action of symethicone disperses and prevents gas bubbles from forming in the gastrointestinal tract.

Therefore, in the light of what disclosed above, it is evident that symethicone is particularly suitable not only for the treatment of gastrointestinal pathologies, flatulency, and colic due to an excess of gases in the gastrointestinal tract, but also for the inhibition and the treatment of bacterial infections of the gastrointestinal apparatus.

As previously mentioned, a synergistic effect between the two components is reached according to the present invention when the concentration of spores of Bacillus Coagulans is between five hundred thousand and fifty thousand millions spores per gram dispersed in a silicone emulsion (symethicone) and particularly when the spore concentration is fifty thousand millions spores per gram.

Bacillus Coagulans is contained in the composition at a concentration between 0.1% and 20% by weight.

Symethicone is contained at a concentration between 5% and 99% by weight.

Optionally, the addition of a food preservative such as sodium benzoate is provided to avoid the pollution by other microbial species.

According to a preferred embodiment the composition according to the present invention includes in 100 grams of final product:

| | |
|---|---|
| • Bacillus Coagulans (fifty thousand millions spores/g) | 10 g |
| • Symethicone PD30 | 89.85 g |
| • Sodium benzoate | 0.15 g |

The composition of the present invention is prepared in the form of liquid solution and/or tablets for oral administering.

Stability tests were executed to test the survival in time of spores of Bacillus Coagulans in the nutrient composition of the present invention. The tests are:
1. vital counting of the spores of Bacillus Coagulans;
2. production capability of lactic acid;
3. challenge test (to strains of Esterichia Coli, Pseudomonas Aeruginosa, Stafilococcus Aureus, Candida Albicans and Aspergillus Niger) to test the preservative capability.

The stability tests at room temperature and at 37°C showed that the vitality of the spores kept in time at high temperature (37°C) even if on a smaller extent than spores at room temperature.

## Claims

1. A composition comprising Bacillus Coagulans and symethicone for use in the treatment of nursling's intestinal disorders.

2. The composition according to claim 1, **characterized in that** symethicone is contained at a concentration by weight between 5% and 99%.

3. The composition according to claim 1, **characterized in that** Bacillus Coagulans are contained at a concentration by weight between 0.1% and 20%.

4. The composition according to claim 2, **characterized in that** the concentration of spores of Bacillus Coagulans is between five hundred thousand and fifty thousand millions spores per gram.

5. The composition according to claim 2, **characterized in that** the concentration of spores of Bacillus Coagulans is fifty thousand millions spores per gram.

6. The composition according to any preceding claim,
**characterized in that** the addition of a food preservative is optionally provided.

7. The composition according to claim 6, **characterized in that** the food preservative is sodium benzoate.

8. The composition according to any preceding claim,
**characterized in that** the composition is prepared in the form of liquid solution for oral administration.

9. The composition according to any claims 1 to 7,
**characterized in that** the composition is prepared in the form of tablets for oral administration.

10. A composition according to any preceding claim,
**characterized in that** the nursling's intestinal disorder is a nursling's gas colic.

11. A composition that can be used for the treatment of the gas colic in nurslings, having the following composition for 100 grams of final product:
| | |
|---|---|
| - Bacillus Coagulans (fifty thousand millions spores/g) | 10 g |
| - Symethicone PD30 | 89.85 g |
| - Sodium benzoate | 0.15 g |

## Patentansprüche

1. Zusammensetzung, umfassend Bacillus coagulans und Simethicon zur Verwendung bei der Behandlung von Intestinalerkrankungen eines Säuglings.

2. Zusammensetzung nach Anspruch 1, **dadurch gekennzeichnet, dass** Simethicon in einer Konzentration von 5 bis 99 Gew.% enthalten ist.

3. Zusammensetzung nach Anspruch 1, **dadurch gekennzeichnet, dass** Bacillus coagulans in einer Konzentration von 0,1 bis 20 Gew.% enthalten ist.

4. Zusammensetzung nach Anspruch 2, **dadurch gekennzeichnet, dass** die Konzentration an Sporen vom Bacillus coagulans fünfhunderttausend bis fünfzigtausend Millionen Sporen pro Gramm beträgt.

5. Zusammensetzung nach Anspruch 2, **dadurch gekennzeichnet, dass** die Konzentration an Sporen von Bacillus coagulans fünfzigtausend Millionen Sporen pro Gramm beträgt.

6. Zusammensetzung nach einem der vorhergehenden Ansprüche, **dadurch gekennzeichnet, dass** wahlweise die Zugabe eines Nahrungsmittel-Konservierungsmittels wahlweise vorgesehen ist.

7. Zusammensetzung nach Anspruch 6, **dadurch gekennzeichnet, dass** das Nahrungsmittel-Konservierungsmittel Natriumbenzoat ist.

8. Zusammensetzung nach einem der vorhergehenden Ansprüche, **dadurch gekennzeichnet, dass** die Zusammensetzung in Form einer flüssigen Lösung zur oralen Verabreichung hergestellt wird.

9. Zusammensetzung nach einem der Ansprüche 1 bis 7, **dadurch gekennzeichnet, dass** die Zusammensetzung in Form von Tabletten zur oralen Verabreichung hergestellt wird.

10. Zusammensetzung nach einem der vorhergehenden Ansprüche, **dadurch gekennzeichnet, dass** die Intestinalerkrankung des Säuglings eine Gaskolik des Säuglings ist.

11. Zusammensetzung, die zur Behandlung der Gaskolik bei Säuglingen verwendbar ist, mit der nachfolgenden Zusammensetzung, bezogen auf 100 Gramm des Endprodukts:
| | |
|---|---|
| - Bacillus coagulans (fünfzigtausend Millionen Sporen/g) | 10 g |
| - Simethicon PD30 | 89,85 g |
| - Natriumbenzoat | 0,15 g |

## Revendications

1. Composition comprenant du Bacillus Coagulans et de la siméthicone pour l'usage dans le traitement des troubles intestinaux du nourrisson.

2. Composition selon la revendication 1,
**caractérisée en ce que** la siméthicone est contenue selon une concentration en poids comprise entre 5% et 99%.

3. Composition selon la revendication 1,
**caractérisée en ce que** la siméthicone est contenue selon une concentration en poids comprise entre 0,1% et 20%.

4. Composition selon la revendication 2,
**caractérisée en ce que** la concentration des spores de Bacillus Coagulans est comprise entre cinq cents mille et cinquante billions de spores par gramme.

5. Composition selon la revendication 2,
**caractérisée en ce que** la concentration des spores de Bacillus Coagulans est égale à cinquante billions de spores par gramme.

6. Composition selon une revendication précédente quelconque, **caractérisée en ce que** l'ajout d'un conservateur alimentaire est prévu de manière optionnelle.

7. Composition selon la revendication 6,
**caractérisée en ce que** le conservateur alimentaire est du benzoate de sodium.

8. Composition selon l'une quelconque des revendications précédentes, **caractérisée en ce que** la composition est préparée sous la forme d'une solution liquide pour l'administration par voie orale.

9. Composition selon l'une quelconque des revendications 1 à 7, **caractérisée en ce que** la composition est préparée sous la forme de comprimés pour l'administration par voie orale.

10. Composition selon l'une quelconque des revendications précédentes, **caractérisée en ce que** les troubles intestinaux des nourrissons sont les coliques flatulentes du nourrisson.

11. Composition pouvant être employée pour le traitement des coliques flatulentes du nourrisson, ayant la composition suivante pour 100 grammes de produit final :
| | |
|---|---|
| - Bacillus Coagulans (cinquante billions de spores/g) | 10 g |
| - Siméthicone PD30 | 89,85 g |
| - Benzoate de sodium | 0,15 g |
